# EUROPEAN PATENT SPECIFICATION

(11) **EP 0 353 707 B1**
(45) Date of publication and mention of the grant of the patent: **22.06.1994**
(21) Application number: 89114181.4
(22) Date of filing: 01.08.1989
(51) Int. Cl.: C07C 69/145, C07C 67/04

(54) **Process for preparing beta-acyloxypropionaldehyde**
Herstellungsverfahren von beta-Acyloxypropionaldehyd
Procédé de préparation de la bêta-acyloxypropionaldéhyde

(30) Priority: 03.08.1988 JP 194221/88
(43) Date of publication of application: 07.02.1990
(73) Proprietor: RESEARCH ASSOCIATION FOR UTILIZATION OF LIGHT OIL, Minato-ku Tokyo (JP)
(72) Inventor: Seto, Takatoshi, Inashiki-gun Ibaraki (JP); Iwane, Hiroshi, Inashiki-gun Ibaraki (JP); Imanari, Makoto, Inashiki-gun Ibaraki (JP)
(74) Representative: Hansen, Bernd, Dr. Dipl.-Chem.

(56) References cited:
- FR-A- 1 307 478
- JP-A-50 005 315

## Description

This invention relates to a process for preparing a β-acyloxypropionaldehyde, and more particularly to a process for preparing a β-acyloxypropionaldehyde by reacting acrolein and a carboxylic acid in the presence of a catalyst.

β-Acyloxypropionaldehyde are useful as a starting material in Strecker's synthesis of homoserine, which is an intermediate material for threonine, one of the useful amino acids. They are also promising as intermediates for synthetic resins, dyes, and plasticizers.

### BACKGROUND OF THE INVENTION

Some cases are known, in which a β-acyloxypropionaldehyde is produced from acrolein and the corresponding carboxylic acid. For example, U.S. Patent 2,857,422 discloses a process of using a strongly basic anion exchange resin as a catalyst, and Mamoru Asao, Kogyo Kagaku Zasshi, Vol. 70, p. 1501, The Chemical Society of Japan, (1967) discloses a process of using magnesium chloride as a catalyst.

These known techniques, however, are not always satisfactory because the catalyst to be used is expensive or the selectivity or yield of the desired product is not sufficiently high.

### SUMMARY OF THE INVENTION

One object of this invention is to provide a process for preparing a β-acyloxypropionaldehyde by using inexpensive catalyst.

Another object of this invention is to provide a process for preparing a β-acyloxypropionaldehyde at improved selectivity and in improved yield.

As a result of extensive studies, the inventors have found that calcium chloride and calcium bromide that are very cheap effectively catalyze the reaction between acrolein and a carboxylic acid to produce a β-acyloxypropionaldehyde at improved selectivity and in improved yield.

That is, the present invention provides a process for preparing a β-acyloxypropionaldehyde comprising reacting acrolein and a carboxylic acid in the presence of calcium chloride and/or calcium bromide as a catalyst.

### DETAILED DESCRIPTION OF THE INVENTION

Starting materials in the process of the present invention are acrolein and a carboxylic acid. The reaction therebetween proceeds as represented by reaction formula (I):

CH₂=CH-CHO + RCO₂H → RCO₂CH₂CH₂CHO (I)

wherein R represents a hydrogen atom, a hydrocarbon group having from 1 to 10 carbon atoms, or a halogenated hydrocarbon group having from 1 to 10 carbon atoms.

Specific examples of the carboxylic acid represented by formula RCO₂H include acetic acid, propionic acid, butyric acid, isobutyric acid, valeric acid, isovaleric acid, benzoic acid, and cyclohexanecarboxylic acid. In view of reaction selectivity, preferred of them are those having a small carbon atom number in R, such as acetic acid and propionic acid.

The starting carboxylic acid is usually used in an amount of from 0.5 to 20 mols, preferably from 2 to 10 mols, per mol of acrolein.

A concentration equilibrium constant Kc of the reaction (I) is represented by equation (II): 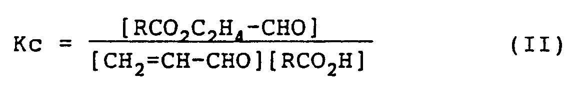
wherein brackets mean molar concentrations of the respective substances.

RCO₂H being made excessive over acrolein in equation (II), the conversion of acrolein to the desired product in the reaction equilibrium state increases.

In the case of carrying out the reaction in a batch system, it is preferable to minimize the amount of unreacted acrolein whose stability is not so high in each batch from the standpoint of working-up procedures for separation, purification, and recovery of the product. To this effect, a charged carboxylic acid/charged acrolein molar ratio of 1 is not always considered optimum from the viewpoint of reaction process since the conversion of acrolein is low.

The optimum reaction selectivity of the catalyst system according to the present invention can be attained with the carboxylic acid/acrolein molar ratio being set slightly higher than employed in the known MgCl₂ catalyst system. Thus, acrolein conversion can be so much increased, leading to a so much improved yield of the desired product.

The acrolein and carboxylic acid to be used as starting materials are not particularly required to have high purity, and small amounts of ordinary organic substances whose acidicity or basicity is around neutrality may be present. It should be noted that strongly basic substances, e.g., sodium hydroxide and methylamine, adversely affect the starting carboxylic acid or the catalyst and that strongly acidic substances, e.g., sulfuric acid, hydrochloric acid, and phosphoric acid, and water adversely affect the starting acrolein or the catalyst. It is therefore desirable that the starting materials should not contain these unfavorable substances.

The catalyst which can be used in the present invention includes calcium chloride, calcium bromide, and a mixture thereof.

Both calcium chloride and calcium bromide embrace a hydrous form and an anhydrous form. Since presence of water tends to reduce selectivity of the reaction (I), the catalyst is preferably used in a water-free form.

The catalyst is usually used in a concentration ranging from 0.1 to 70% by weight, preferably from 1 to 30% by weight.

As long as the catalytic activity of the catalyst is not impaired, additives may be added to the catalyst of the present invention. The additives include alkali metal halides, e.g., KCl and KBr, alkaline earth metal halides, e.g., BaCl₂ and BaI₂, and rare earth metal halides, e.g., LaCl₃ and CeCl₃.

Since the catalyst according to the present invention does not undergo denaturation of its inorganic component during the reaction, it can withstand a repeated use.

The reaction is carried out in an inert gas atmosphere in order to prevent oxidation of acrolein. In cases where the reaction is performed under conditions which makes the reaction time relatively long, it is preferable to incorporate a polymerization inhibitor of acrolein, such as hydroquinone, into the reaction system. Hydroquinone, if added, is used in an amount of from 0.002 to 2% by weight based on acrolein. Larger amounts of hydroquinone than necessary tend to reduce the yield of the desired product and the reaction selectivity.

The reaction can be carried out in the presence of a solvent. Since there is a limit in solubility of CaCl₂ or CaBr₂ in the reaction mixture comprising acrolein and a carboxylic acid, and the insoluble content of the catalyst exhibits almost no catalytic activity, addition of a solvent to increase the solubility of the catalyst is effective to increase the reaction rate. Solvents usable to this effect include dimethyl sulfoxide and dimethylformamide. In such a solvent system, when applied to a batchwise reaction, the concentration of the dissolved catalyst per batch can be increased so that the reaction time per batch can be shortened. Additionally included in the solvents which can be used in this invention are tetrahydrofuran, dioxane, pyrrolidone, 2,6-dimethylpyridine, pyridazine, quinoline, triazole, oxazole, pyrazine, pyrazole, phenol, cresol.

The reaction represented by reaction formula (I) per se can be effected in any of a gaseous phase, a liquid phase, and a gas-liquid mixed phase, and the catalyst to be employed may be either of a homogeneous system or of a heterogeneous system. However, since the insoluble portion of the calcium catalyst according to the present invention exhibits substantially no activity, it is preferable to carry out the reaction in a liquid phase in a homogeneous system.

The reaction is conducted in an inert gas atmosphere in order to prevent undesired reactions, such as oxidation and polymerization of acrolein. The inert gas to be used includes nitrogen, argon, and carbonic acid gas. The reaction temperature usually ranges from -80 to 150°C, preferably from -20 to 90°C.

After completion of the reaction, the desired product, a β-acyloxypropionaldehyde, can be easily be isolated and purified by known techniques, such as distillation. The unreacted acrolein and carboxylic acid can be recovered and returned to the reaction system.

The present invention is now illustrated in greater detail with reference to the following Examples, but it should be understood that the present invention is not deemed to be limited thereto. In these examples, all the percents are by weight unless otherwise indicated.

### EXAMPLE 1

Into a 300 ml-volume glass autoclave whose atmosphere had been displaced with nitrogen were charged 126.12 g (2.10 mol) of acetic acid of first grade (produced by Wako Pure Chemical Industries, Ltd.), 22.2 g (0.376 mol) of acrolein (containing 0.1 to 0.25% of a polymerization inhibitor; purity: 95%; produced by Tokyo Chemical Industry Co., Ltd.), and 12.21 g (0.110 mol) of anhydrous calcium chloride of first grade (produced by Wako Pure Chemical Industries, Ltd.), and the mixture was stirred at 40°C for 16 hours to effect reaction. During the reaction, the calcium chloride was in a completely dissolved state. After the reaction, the reaction mixture was analyzed by gas chromatography. The reaction product isolated from the reaction mixture was identified to be β-acetoxypropionaldehyde by NMR, IR and mass spectra and elementary analysis. The conversion of the starting acrolein was 63.1%, and the selectivity was 97.6%.

### EXAMPLE 2

The procedure of Example 1 was repeated, except that 21.99 g (0.110 mol) of anhydrous calcium bromide (prepared by dehydrating CaBr₂·6H₂O, an EP reagent produced by Nakarai Kagaku Yakuhin K.K., by evacuation under heating to obtain a di- to trihydrate and then dehydrating in a mixed stream of hydrogen and hydrogen bromide) was used as a catalyst in a place of calcium chloride and the reaction was continued for 20 hours.

The acrolein conversion was 61.8%, and the selectivity of β-acetoxypropionaldehyde was 91.2%.

### EXAMPLE 3

The procedure of Example 1 was repeated, except for changing the amount of acrolein to 32.81 g (0.556 mol as pure acrolein) and changing the amount of acetic acid to 114.49 g (1.907 mol).

The acrolein conversion was 60.4%, and the selectivity to β-acetoxypropionaldehyde was 95.5%.

### EXAMPLE 4

The reaction mixture obtained in Example 1 was transferred to a flask and distilled under reduced pressure at a bottom temperature of 50°C or lower to remove any unreacted acrolein, unreacted acetic acid, and the produced β-acetoxypropionaldehyde. The remaining catalyst residue at the bottom was returned to the glass autoclave. The same starting materials of the same amounts were charged in Example 1 except for the catalyst in the autoclave, and the reaction was effected under the same conditions as in Example 1.

The acrolein conversion was found to be 60.2%, and the selectivity to β-acetoxypropionaldehyde was found to be 98.6%.

### COMPARATIVE EXAMPLE 1

The procedure of Example 1 was repeated, except for replacing calcium chloride with 12.21 g of anhydrous magnesium chloride (produced by Wako Pure Chemical Industries, Ltd.) as a catalyst and changing the reaction time to 11 hours.

The acrolein conversion was 60.9%, and the selectivity to β-acetoxypropionaldehyde was 88.3%.

### COMPARATIVE EXAMPLE 2

The procedure of Comparative Example 1 was repeated, except for changing the amounts of acrolein, acetic acid, and anhydrous magnesium chloride to 66.82 g (1.192 mol as pure acrolein), 78.0 g (1.299 mol), and 15.08 g (0.158 mol), respectively, and changing the reaction time to 7 hours.

The acrolein conversion was 58.2%, and the selectivity to β-acetoxypropionaldehyde was 92.1%.

### COMPARATIVE EXAMPLE 3

The procedure of Example 1 was repeated, except for using 67.26 g of a strongly basic anion exchange resin "Amberlite IRA 400" produced by Rhom & Haas Co. [having been subjected to the treatment described in Mamoru Asao, Kogyo Kagaku Zasshi, Vol. 70, No. 9, p. 1502, The Chemical Society of Japan, (1967)] as a catalyst.

The acrolein conversion was 59.6%, and the selectivity to β-acetoxypropionaldehyde was 90.5%.

As described above, the present invention, in which acrolein and a carboxylic acid are reacted using a very cheap calcium halide as a catalyst, makes it possible to prepare a β-acyloxypropionaldehyde at higher conversions and selectivities than reached in conventional reaction system using magnesium chloride or strongly basic ion exchange resins. In addition, the catalyst and unreacted starting materials used in the present invention can be recycled to the reaction system. Accordingly, the present invention produces extremely useful effects in production of β-acyloxypropionaldehydes on an industrial scale.

## Claims

1. A process for preparing a β-acyloxypropionaldehyde comprising reacting acrolein and a carboxylic acid in the presence of calcium chloride and/or calcium bromide as a catalyst.

2. A process as claimed in claim 1, wherein said carboxylic acid is used in an amount of from 0.5 to 20 mols per mol of acrolein.

3. A process as claimed in claim 1, wherein said carboxylic acid is used in an amount of from 2 to 10 mols per mol of acrolein.

4. A process as claimed in claim 1, wherein said calcium chloride and/or calcium bromide is/are in an anhydrous form.

5. A process as claimed in claim 1, wherein said calcium chloride and/or calcium bromide is/are present in a reaction mixture in a total amount of from 0.1 to 70% by weight.

6. A process as claimed in claim 1, wherein said calcium chloride and/or calcium bromide is/are present in a reaction mixture in a total amount of from 1 to 30% by weight.

7. A process as claimed in claim 1, wherein said reacting is in a liquid phase in the presence of a solvent.

8. A process as claimed in claim 7, wherein said solvent is dimethyl sulfoxide or dimethylformamide.

## Patentansprüche

1. Verfahren zur Herstellung eines beta-Acyloxypropionaldehydes, umfassend die Reaktion von Acrolein und einer Carbonsäure in der Gegenwart von Kalziumchlorid und/oder Kalziumbromid als ein Katalysator.

2. Verfahren nach Anspruch 1, worin die Carbonsäure in einer Menge von 0,5 bis 20 Mol pro Mol Acrolein verwendet wird.

3. Verfahren nach Anspruch 1, worin die Carbonsäure in einer Menge von 2 bis 10 Mol pro Mol Acrolein verwendet wird.

4. Verfahren nach Anspruch 1, worin das Kalziumchlorid und/oder Kalziumbromid in wasserfreier Form vorliegt.

5. Verfahren nach Anspruch 1, worin das Kalziumchlorid und/oder Kalziumbromid in einer Reaktionsmischung in einer Gesamtmenge von 0,1 bis 70 Gew.% vorhanden ist.

6. Verfahren nach Anspruch 1, worin das Kalziumchlorid und/oder Kalziumbromid in einer Reaktionsmischung in einer Gesamtmenge von 1 bis 30 Gew.% vorhanden ist.

7. Verfahren nach Anspruch 1, worin die Reaktion in einer Flüssigphase in der Gegenwart eines Lösungsmittels durchgeführt wird.

8. Verfahren nach Anspruch 7, worin das Lösungsmittel Dimethylsulfoxid oder Dimethylformamid ist.

## Revendications

1. Procédé de préparation d'un β-acétoxypropionaldéhyde comprenant la réaction de l'acroléine et d'un acide carboxylique en présence de chlorure de calcium et/ou de bromure de calcium comme catalyseur.

2. Procédé selon la revendication 1, dans lequel ledit acide carboxylique est utilisé en une quantité comprise entre 0,5 et 20 moles par mole d'acroléine.

3. Procédé selon la revendication 1, dans lequel ledit acide carboxylique est utilisé en une quantité comprise entre 2 et 10 moles par mole d'acroléine.

4. Procédé selon dans la revendication 1, dans lequel ledit/lesdits chlorure de calcium et/ou bromure de calcium est/sont sous forme anhydre.

5. Procédé selon la revendication 1, dans lequel ledit/lesdits chlorure de calcium et/ou bromure de calcium est/sont présent(s) dans le mélange réactionnel en une quantité totale comprise entre 0,1 et 70 % en poids.

6. Procédé selon la revendication 1, dans lequel ledit/lesdits chlorure de calcium et/ou bromure de calcium est/sont présent(s) dans un mélange réactionnel en une quantité totale comprise entre 1 et 30 % en poids.

7. Procédé selon la revendication 1, dans lequel ladite réaction se produit dans une phase liquide en présence d'un solvant.

8. Procédé selon la revendication 7, dans lequel ledit solvant est du diméthylsulfoxyde ou du diméthylformamide.
